# EUROPEAN PATENT APPLICATION

(11) **EP 4 154 925 A1**
(43) Date of publication of application: **29.03.2023**
(21) Application number: 21198395.2
(22) Date of filing: 22.09.2021
(51) Int. Cl.: A61M 1/34

(54) **IMMUNE CELL TREATMENT**

(71) Applicant: Artcline GmbH, 18057 Rostock (DE)
(72) Inventor: ALTRICHTER, Jens, 18057 Rostock (DE)
(74) Representative: Uexküll & Stolberg

(57) **Abstract**

The present invention generally relates to the field of blood treatment. More particularly, the invention relates to an *ex vivo* method for treating an immunological dysfunction of blood. The invention also relates to an *ex vivo* method for removing toxic or inflammatory compounds from blood plasma and/or supplementing blood plasma with immunologically active compounds. The methods of the invention comprise the steps of extracorporeally separating at least part of the plasma from the blood and conducting the separated plasma through a plasma-permeable hollow fiber filter with immune cells located thereon such that the plasma comes into contact with the immune cells. The invention also relates to the use of a plasma-permeable hollow fiber filter with immune cells located thereon for the *ex vivo* treatment of an immunological dysfunction of blood. Finally, the present invention relates to the use of a plasma-permeable hollow fiber filter with immune cells located thereon for removing toxic or inflammatory compounds from blood plasma and/or supplementing blood plasma with immunologically active compounds.

## Description

The present invention generally relates to the field of blood treatment. More particularly, the invention relates to an *ex vivo* method for treating an immunological dysfunction of blood. The invention also relates to an *ex vivo* method for removing toxic or inflammatory compounds from blood plasma and/or supplementing blood plasma with immunologically active compounds. The methods of the invention comprise the steps of extracorporeally separating at least part of the plasma from the blood and conducting the separated plasma through a plasma-permeable hollow fiber filter with immune cells located thereon such that the plasma comes into contact with the immune cells. The invention also relates to the use of a plasma-permeable hollow fiber filter with immune cells located thereon for the *ex vivo* treatment of an immunological dysfunction of blood. Finally, the present invention relates to the use of a plasma-permeable hollow fiber filter with immune cells located thereon for removing toxic or inflammatory compounds from blood plasma and/or supplementing blood plasma with immunologically active compounds.

### BACKGROUND OF THE INVENTION

Sepsis and septic shock remain major causes of morbidity and mortality in critically ill patients and the treatment of these patients is very expensive [1-4]. The impairment of hemodynamics and liver function are major problems in patients with sepsis [5, 6]. In patients with septic shock a liver dysfunction or liver failure occurred in nearly 19% and lead to a poor prognosis of these patients [6, 7].

Extracorporeal therapies have been suggested to influence successfully immune imbalances and subsequently the clinical course of multiorgan failure and sepsis [8]. Some studies showed hemodynamic stabilization of patients during extracorporeal treatment of sepsis; however, no clear impact on survival was seen [8, 9]. The influence of extracorporeal therapies of sepsis on liver function has not been investigated yet, but it may be an important tool for the improvement of outcome in this high-risk cohort population with liver dysfunction or liver failure [6]. Apart from the above, extracorporeal bioreactors were studied in the treatment of liver failure and acute renal failure associated with sepsis using hepatocytes or renal tubular cells; the proper choice of the cell-source was of central importance [10-14].

The use of immune cells (leukocytes) to treat sepsis in an extracorporeal setting was also reported [15]. With regard to cellular immunocompetence, functional impairment of neutrophils and monocytes is associated with increased mortality in advanced stages of sepsis [16-23]. Therefore, an extracorporeal immune cell bioreactor system was developed [24, 25], mainly containing granulocytes in circulation. The rationale for such an approach is that on one hand the plasma-modifying capacity of human phagocytes can be used (e.g., to remove antigenic material from the circulation) while on the other hand control over the immune cells can be maintained (e.g., retention of the immune cells and their release and break-down products, preventing local tissue effects; 15). In *in vitro* studies and two animal models for septic shock, promising survival data were obtained [15, 24, 26]. Additionally, the immune cell bioreactor was already tested with septic shock patients and showed safety and compatibility of this complex therapy [25].

Another suitable method for the extracorporeal treatment of sepsis is described in international patent application WO 2009/103553 A1. The method is essentially based on an extracorporeal blood circuit and a cell circuit, which are connected by two filters. In the extracorporeal blood circuit, the plasma is separated from the patient's blood by use of special plasma separators, e.g. a plasma filter. The separated plasma is then transferred to a second circuit containing the immune cells of a healthy blood donor. Here, the patient plasma comes into direct contact with the donor immune cells that circulate in the second circuit. These cells bind and remove bacterial toxins as well as the waste products that occur in the plasma. Furthermore, the immune cells react to signalling molecules present in the plasma and release various immunologically active molecules, e.g. cytokines. The treated plasma is then separated from the donor immune cells by a second plasma filter and returned to the patient together with the patient's blood. There, the immunologically active molecules are supposed to reactivate the patient's own immune system so that, on the one hand infections are effectively fought and, on the other hand organ dysfunctions normalise. The treatment of the patient's plasma takes place continuously over several hours.

A drawback of the method described in WO 2009/103553 A1 resides in the fact that a process with two circuits requires a high material outlay. Moreover, such a process is demanding in terms of handling. In addition, the immune cells, e.g. the granulocytes, are subjected to a high level of stress that results from the continuous movement of the cells through the tubing which may affect their effectiveness and viability. Accordingly, there is still a need for an improved method for the extracorporeal treatment of septic blood that is easy to handle and provides a more gentle environment to the immune cells used. The present invention addresses this need and provides additional advantages as well.

### DESCRIPTION OF THE INVENTION

It has been surprisingly found in the course of the present invention that the continuous movement of the immune cells is not an absolute requirement for retaining the activity of the cells. Specifically, as shown in the below examples, it is possible to provide the immune cells on or within a plasma-permeable hollow fiber filter without interfering with their detoxifying activity or their ability to secrete cytokines and other effector molecules into the plasma. By using immune cells attached to the surface of a plasma-permeable hollow fiber filter, the necessity of circulating the cells is eliminated.

Thus, in a first aspect, the present invention provides an ex vivo method for the treatment of an immunological dysfunction of blood, said method comprising the steps of:
(a) extracorporeally separating at least part of the plasma from blood obtained from a patient suffering from an immunological dysfunction of the blood,
(b) conducting the separated plasma through a plasma-permeable hollow fiber filter comprising immune cells located thereon such that the plasma comes into contact with the donor immune cells, and
(c) returning the plasma obtained from step (b) to the blood.

In a second aspect, the present invention provides an *ex vivo* method for the removal of toxic or inflammatory compounds from blood plasma and/or the supplementation of blood plasma with immunologically active proteins, said method comprising the steps of:
(a) extracorporeally separating at least part of the plasma from blood obtained from a patient,
(b) conducting the separated plasma through a plasma-permeable hollow fiber filter comprising immune cells located thereon such that the plasma comes into contact with the immune cells, and
(c) returning the plasma obtained from step (b) to the blood.

The above methods are based on the direct contact of immune cells, such as immune cells from a healthy donor, with plasma derived from a patient. Accordingly, the methods of the invention are directed to a plasma perfusion method. Thus, in a third aspect, the invention provides an *ex vivo* method for plasma perfusion comprising the steps of:
(a) extracorporeally separating at least part of the plasma from blood obtained from a patient,
(b) conducting the separated plasma through a plasma-permeable hollow fiber filter comprising immune cells located thereon such that the plasma comes into contact with the donor immune cells, and
(c) returning the plasma obtained from step (b) to the blood.

In a fourth aspect, the present invention provides an *ex vivo* method for producing a blood sample that is depleted of certain toxic or inflammatory compounds and/or supplemented with other immunologically active proteins, said method comprising the steps of:
(a) extracorporeally separating at least part of the plasma from blood obtained from a patient,
(b) conducting the separated plasma through a plasma-permeable hollow fiber filter comprising immune cells located thereon such that the plasma comes into contact with the immune cells, and
(c) returning the plasma obtained from step (b) to the blood.

The above methods are performed with a blood sample that has been obtained from a patient, e.g. by venipuncture or from an arterial or venous catheter, like a Shaldon catheter. The patient may suffer from an immunological dysfunction of the blood which means that certain immunological functions provided by blood of a healthy individual are compromised in the blood of said patient. For example, the blood may lack a sufficient number of macrophages or other blood cells that are responsible for or involved in phagocytosis, or the function of said cells may be impaired so that bacteria and other pathogens cannot be removed effectively from the blood.

In a preferred embodiment, the blood is derived from a patient suffering from sepsis or a septic shock. During sepsis, an immunological dysfunction of blood components has been reported. Sepsis is characterized by an initial hyperinflammatory phase and a subsequent immunosuppressive phase. In the initial phase, cytokines and other effector molecules are secreted which influence both phagocytosis and downstream signalling processes. The later immunosuppressive phase is characterized by a reduced functionality of phagocytes and antigen-presenting cells and a reduced number of effector cells. This phase, which is also referred to as immunoparalysis or compensatory anti-inflammatory response syndrome (CARS) in the literature, is associated with an increased mortality in advanced stages of sepsis and septic shock [29, 30, 31].

In step (a) of the above methods, at least part of the plasma obtained from a patient, e.g. a patient suffering from an immunological dysfunction of the blood, is extracorporeally separated from the blood. Conveniently, the separation of the plasma can be achieved by running the blood through a plasma-permeable filter. Suitable plasma filters are obtainable from different manufacturers, such as the Haemoselect^{®} 0.5/0.7 plasma filters (B Braun, Melsungen, Germany), the PF1000 or PF2000 plasma filters (Gambro Lundia AB / Baxter, Lund, Sweden), the Granopen plasma filters (Infomed SA, Meinier, Switzerland), the Versatile^{®}-PES Plasmart plasma filter series or SepaPlas plasma filter series e.g. SepaPlas 06 (Medica S.p.A., Medolla, Italy). These filters are permeable for the plasma while retaining blood cells.

The separated plasma is then led through a plasma-permeable hollow fiber filter having immune cells located thereon, preferably immune cells from a healthy donor. As used herein, a hollow fiber filter refers to a filter matrix that comprises a high number of tubes which resemble tiny straws and are tightly bundled together. The walls of the tubes contain microscopic pores or holes which are permeable for the plasma, but not permeable for larger particles or cells. The tubes of the hollow fiber filter are made of a polymeric material such as polyethersulfone and are normally packed into a cartridge with an outer rigid housing. The hollow fiber filter cartridge normally comprises an inlet, an outlet and one or more side ports. This type of filter cartridge is routinely used for tangential flow filtration (also known as cross-flow filtration). In this tangential flow filtration approach, a feed stream enters the filter cartridge via an inlet and travels tangentially across the surface of the hollow fibers before it leaves the filter cartridge via an outlet. A part of the plasma passes through the membrane as permeate or filtrate and leaves the cartridge via a side port, while the remaining plasma and cells are retained on the feed side of the membrane as retentate.

According to the invention, the plasma-permeable hollow fiber filter is used for a dead-end filtration. This means that the feed stream is led through the filter instead of tangentially across the surface of the filter. This can be achieved, in one embodiment, by using a standard hollow fiber cartridge having an inlet, an outlet and one or more side ports and adapting the filter such that only the inlet and one side port are kept open while the outlet and the remaining side ports are closed. As a standard cartridge is normally equipped with covers or caps for the inlet, the outlet and the side ports, a closure of the outlet and the side ports can be achieved by simply sealing them with the respective covers or caps which are provided by the manufacturer. Alternatively, these caps are available as sterile spare parts. The plasma and the cells may then be led into the filter cartridge via the inlet, and the plasma passes through the filter and leaves the filter via the side port. In contrast, the cells are unable to pass through the filter and remain within the inner lumen of the hollow fibers.

Alternatively, in another embodiment, the cells and the plasma may also let through the side port into the filter cartridge. The plasma will then pass through the outer surface of the hollow fibers and enter into the lumen of the hollow fibers. The plasma will then leave the cartridge via the open outlet, while the cells remain on the outer surface of the hollow fibers. This latter approach may be particularly useful for applications in which a larger filter surface is required, e.g. when high volumes of plasma or larger cell numbers are to be filtrated.

The plasma is contacted with the hollow fiber filter under conditions that allow the plasma to come into direct contact with the immune cells. In particular, filtration pressure and flow rate/velocity will be selected such that the plasma flows around the cells located on or within the filter for a sufficient time before passing the filter to allow the cells to remove toxic or inflammatory compounds or cell debris from the plasma and/or to secrete cytokines and other immunologically active proteins into the plasma.

The immune cells may be located on the outer surface of the hollow fibers or within the fibers of the hollow fiber filter. Preferably, the immune cells are located within the fibers of the hollow fiber filter. This means that the cells enter into the hollow fibers and become entrapped within these fibers during the filtration process. Unexpectedly, this does not affect the ability of the cells to remove toxic or inflammatory compounds from the plasma stream, and it also does not interfere with cytokine production and secretion, as shown in the example part below.

The immune cells to be used in the methods of the invention preferably comprise granulocytes, monocytes, macrophages and/or lymphocytes. As used herein, granulocytes refer to a subtype of leukocytes which make up the immune system's main cellular defense against bacterial and fungal infections. The group of granulocytes can be divided into neutrophils, eosinophils and basophils. Neutrophils are particularly important for the innate immunity. Their main functions are phagocytosis and elimination of microorganisms. The normal physiological half-life of granulocytes is 5-9 h. The term group of lymphocytes comprises T cells, B cells and natural killer (NK) cells.

The granulocytes, monocytes, macrophages and/or lymphocytes for use in the methods of the invention may originate from different sources. In one embodiment, the granulocytes, monocytes, macrophages and/or lymphocytes are derived from a healthy donor. In another embodiment, the granulocytes, monocytes, macrophages and/or lymphocytes are derived from undifferentiated progenitor cell lines, such as stem cells. While the methods of the invention are not particularly limited with regard to the type of granulocytes, monocytes, macrophages and/or lymphocytes, it is preferred that these cells are mammalian cells, more preferably human cells. In the particularly preferred embodiment, the methods of the invention are performed with human granulocytes, monocytes, macrophages and/or lymphocytes, more preferably human granulocytes and/or lymphocytes of the healthy human donor.

In a particularly preferred embodiment, the immune cells used in the methods of the invention are derived from a granulocyte concentrate. Granulocyte concentrates may be obtained from the blood of donors by commonly known methods, such as apheresis. The donors may be treated with corticosteroids or recombinant growth factors, such as granulocyte colony-stimulating factor (G-CSF) prior to apheresis to increase granulocyte yield and prolong granulocyte half-life. To increase the quality of the granulocyte concentrate, the blood collected from the donor may be supplemented with an additive like high-molecular-weight hydroxyethyl starch (HES), gelatine or dextrane which facilitates sedimentation prior to apheresis, thereby allowing a better separation of granulocytes from erythrocytes. Conventional granulocyte concentrates usually contain 5-10% granulocytes while the majority of the cells are erythrocytes or platelets.

It has been shown that the methods of the invention are particularly efficient if the immune cells provided on the hollow fiber filter have been enriched for granulocytes. For example, the methods of the invention are particularly efficient if the population of immune cells used on the hollow fiber filter comprise at least 10% granulocytes, at least 20% granulocytes, at least 30% granulocytes, at least 40% granulocytes, at least 45% granulocytes, at least 50% granulocytes, at least 55% granulocytes, at least 60% granulocytes, at least 75% granulocytes, at least 90% granulocytes, or at least 99% granulocytes relative to the overall number of cells which are present on the hollow fiber filter.

Preferably, the filter comprises from 1 x 10⁹ to 1 x 10¹² granulocytes, more preferably from 1 x 10⁹ to 1 x 10¹¹, more preferably from 1 x 10¹⁰ to 1 x 10¹¹, and even more preferably from 1.2 x 10¹⁰ to 2.5 x 10¹⁰ granulocytes. For example, the number of granulocytes which are present on the hollow fiber filter may be at least 1 x 10⁹, at least 5 x 10⁹, at least 1 x 10¹⁰, at least 5 x 10¹⁰, at least 1 x 10¹¹, or at least 5 x 10¹¹. A filter with about 1.2 x 10¹⁰ to 2.5 x 10¹⁰ granulocytes located thereon is particularly preferred for use in the methods of the invention.

Apart from the granulocytes, the hollow fiber filter may also comprise other leukocytes, preferably in the amount from 1 x 10⁸ to 1 x 10¹⁰, more preferably from 1 x 10⁹ to 1 x 10¹⁰, more preferably from 5 x 10⁹ to 1 x 10¹⁰. For example, the number of other leukocytes which are present on the hollow fiber filter may be at least 1 x 10⁸, at least 5 x 10⁸, at least 1 x 10⁹, or at least 5 x 10⁹. A filter with about 1 x 10¹⁰ or about 1 x 10⁹ other leukocytes located thereon is particularly preferred for use in the methods of the invention.

According to the invention, it has been found to be advantageous to take measures that provide for a reduced number of erythrocytes on the hollow fiber filter. A limited number of erythrocytes prevents clogging of the filter and ensures a constant filtration performance. It is hence preferred to perform the methods of the invention with a limited number of erythrocytes on the hollow fiber filter. Preferably, the number of erythrocytes which are present on the hollow fiber filter is less than 1 x 10¹¹ erythrocytes, more preferably less than 2.04 x 10¹⁰ erythrocytes, and even more preferably less than 1 x 10⁶ erythrocytes.

Stated differently, it is preferred that less than 50% of the cells located on the hollow fiber filter are erythrocytes. More preferably, less than 40% of the cells on the filter are erythrocytes, and even more preferably less than 30%, less than 25%, less than 20%, less than 15%, less than 10%, less than 5%, less than 4%, less than 3%, less than 2%, or less than 1% of the cells. A suitable method for concentrating granulocytes and depleting erythrocytes is disclosed, for example, in international application WO 2013/182311 A1.

The cells are located on or within the hollow fiber filter and remain there during the filtration step (b) of the above methods. In the particularly preferred embodiment, the methods of the present invention do not comprise a circulation of the immune cells. Instead, the cells remain stationary adhered to the hollow fiber filter during step (b) of the above methods. It has been surprisingly found in the course of the present invention that granulocytes which have been enriched or even purified by the depletion of erythrocytes are more prone to adhering to the surface of a filter of filter membrane compared to granulocytes in normal blood samples. Accordingly, the use of granulocyte preparations which have been enriched, concentrated or purified in the methods of the invention is particularly preferred.

In a preferred embodiment, the cells are located either on the outer surface of the hollow fiber filter or at the inner surface of the hollow fibers. In a particularly preferred embodiment, the cells are located at the inner surface of the hollow fibers which means that the cells enter the fibers with the feed stream, i.e. the plasma stream, and remain within the fibers while the cell-free plasma leaves the filter as the permeate. It is also possible that the cells have been led into the filter via the inlet before the plasma is led through the filter. It has been surprisingly found that the accumulation of the immune cells within the hollow fibers does not disturb or otherwise interfere with the immunological function of the cells in phagocytosis and the exchange of signaling compounds with the plasma which flows through the filter. This insight allows the use of a high number of cells in accumulated form on the filter for plasma perfusion.

The hollow fiber filter has an average membrane pore size which is small enough to ensure that the immune cells cannot pass the filter. Preferably, the average pore size of the hollow fiber filter will be 0.8 µm or less, more preferably 0.5 µm or less, and more preferably 0.2 µm or less. A pore size of about 0.5 µm is particularly preferred for use in the methods of the present invention. The hollow fiber filter can be either coated or uncoated and consist of a number of different materials including, but not limited to polysulfone, polyethersulfone, polyamide, polymethyl methacrylate, polystyrene, polycarbonate, polyethylene (PE), polytetrafluoroethylene (PTFE), polypropylene (PP), or nylon. A preferred hollow fiber filter for use in the methods of the invention, for example, consists of polypropylene or polyethersulfone. An effective membrane surface area in the range from 0.1 to 1 m², such as about 0.2 m², about 0.3 m², about 0.4 m², about 0.5 m², about 0.6 m², about 0.7 m², about 0.8 m², or about 0.2 m². A polyethersulfone hollow fiber filter with an effective membrane surface area in the range from 0.5 to 0.7 m², is particularly preferred.

Since the immune cells are deposited on the surface of the filter, it is particularly preferred that the filter is biocompatible, i.e., non-toxic for the immune cells, e.g. granulocytes and or lymphocytes, deposited thereon. To increase biocompatibility, the filter can be coated, for example with one or more biologically active substances that prevent coagulation or promote cell attachment. The filter may further be coated with biologically inert substances, such as metals or ceramics.

The filtration step (b) of the methods of the present invention is preferably conducted as a dead-end filtration. In this type of filtration, a particle- or cell-containing fluid is pumped against the filter at low pressure to minimize the compaction of the retained particles or cells. Due to the permanent outflow of the filtrate, the particles or cells to be separated accumulate on the surface of the filter.

According to the invention, the plasma is obtained from filtration (b) step using the hollow fiber filter can be passed through a third filter that acts as a safety filter which removes residual cells that may still be comprised in the filtered plasma e.g. due to a membrane rupture of said plasma-permeable hollow fiber filter. The third filter may be another hollow fiber filter or any other filter type that is routinely used for cell removal, e.g. a filter which is used for the sterile filtration of liquids. Therefore, the safety filter may be of the same type or a different type compared first plasma-permeable hollow fiber filter.

In one embodiment, the temperature of the plasma is controlled during the filtration process. For example, the plasma may be heated to a temperature of between 35°C and 41°C, preferably 37°C, before or after passing through the hollow fiber filter. Similarly, the temperature of cells on the hollow fiber filter is controlled during the filtration process. For example, the filter and the cells may be heated to a temperature of between 35°C and 38°C in order to maintain the viability of the cells.

In a fifth aspect, the invention relates to the use of a plasma-permeable hollow fiber filter with immune cells located thereon for the *ex vivo* treatment of an immunological dysfunction of blood. The hollow fiber filter is a filter as described herein in connection with the first, second and third aspect of the invention. Similarly, the immune cells are cells as described herein in connection with the first, second, third and fourth of the invention.

In a sixth aspect, the invention relates to the use of a plasma-permeable hollow fiber filter with immune cells located thereon for removing toxic or inflammatory compounds from blood plasma and/or supplementing blood plasma with immunologically active proteins. The hollow fiber filter is a filter as described herein in connection with the first, second, third and fourth aspect of the invention. Similarly, the immune cells are cells as described herein in connection with the first, second and third aspect of the invention.

In a seventh aspect, the invention relates to the use of a plasma-permeable hollow fiber filter with immune cells located thereon for plasma perfusion. The hollow fiber filter is a filter as described herein in connection with the first, second and third aspect of the invention. Similarly, the immune cells are cells as described herein in connection with the first, second, third and fourth aspect of the invention.

In a eighth aspect, the invention pertains to the use of a plasma-permeable hollow fiber filter with immune cells located thereon for producing a blood sample depleted of certain toxic or inflammatory compounds and/or supplemented with other immunologically active proteins.

In an ninth aspect, the invention pertains to a device for carrying out the methods of the first, second, third and fourth aspect of the invention, said device comprising a plasma-permeable hollow fiber filter with immune cells located thereon.

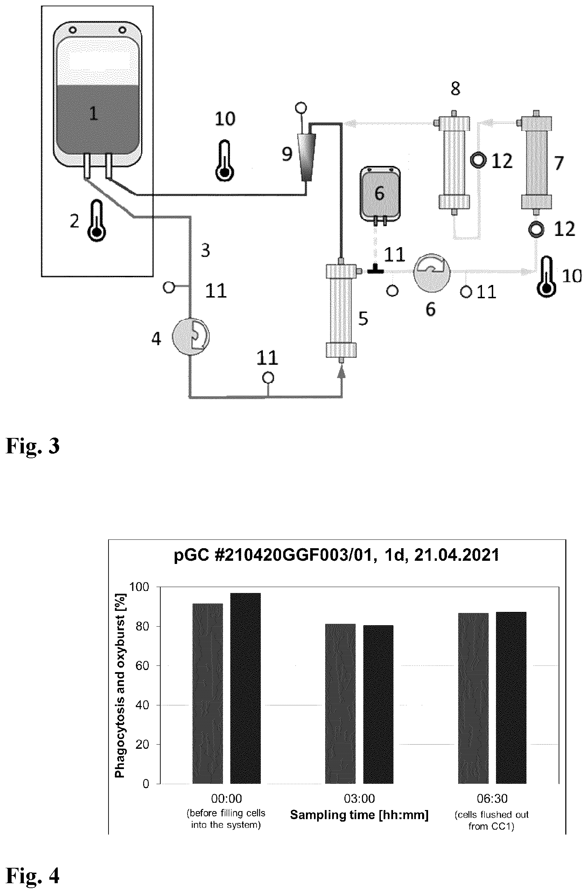

Figure 3 shows a device suitable for conducting the method of the present invention. The device comprises a plasma pool 1 which can be a plastic bag that has a volume of 500-1000 mL and comprises fresh frozen donor plasma (FFP). The plasma pool is kept at a temperature of 37°C by heating device 2. The plasma is passed through the sterile tubing 3 via a first pump 4 and enters filter 5, where part of the plasma is separated. The plasma is mixed with the immune cells which are encompassed in the cell reservoir 6, and the mixture is transported by pump 6 to a second filter 7 which is a hollow fiber filter that holds back the immune cells. Once the cells have attached to the hollow fiber filter, the plasma perfuses filter 7 with the cells attached thereto in a dead-end filtration mode. Subsequently, the plasma is led through another filter 8 which acts as a safety barrier in case of a membrane rupture in filter 7 to avoid cells entering the circuit. Finally, the plasma which has been in contact with the immune cells is led into venous chamber 9 where it is heated again to 37°C by heating device 10, before it is combined again with the blood components from filter 5 before it is returned to the plasma pool. The tubing 3 is connected with pressure tranducers 11 and sample ports 12, the latter of which allow for the withdrawal of samples that can be tested for certain parameters.

### BRIEF DESCRIPTION OF THE FIGURES

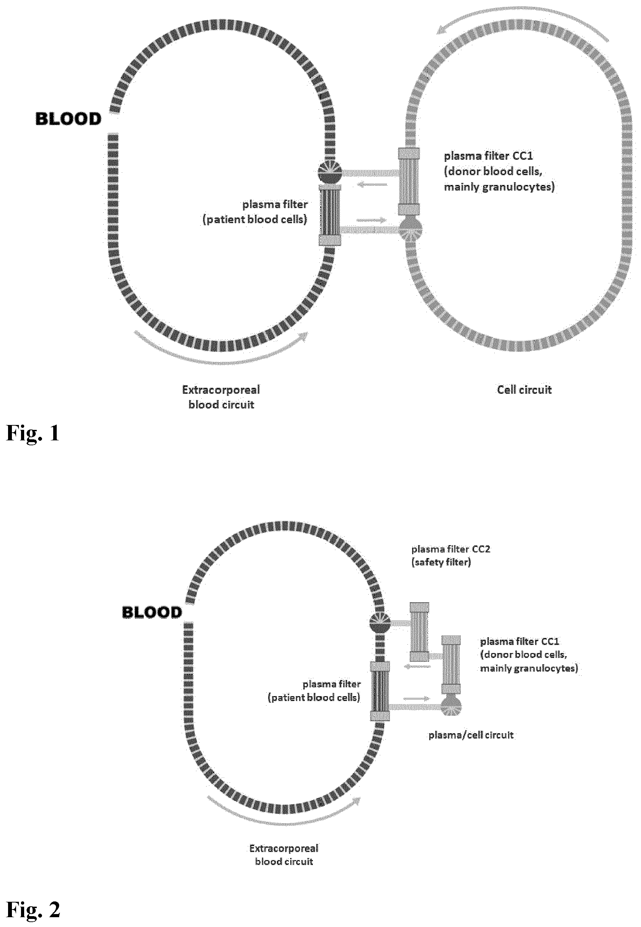

**Figure 1** shows the method of granulocyte perfusion known in the art which comprises the separation of plasma from an extracorporeal blood circuit by a plasma filter and feeding said plasma into a second circuit of circulating granulocytes. The granulocytes provide for the removal of toxic compounds from the plasma and secreting cytokines and other immunologically active proteins into the plasma.
**Figure 2** shows the immune cell perfusion method according to the invention in which plasma from an extracorporeal blood circuit is separated by a plasma filter and led through a hollow fiber filter with donor immune cells that adhere to the filter, preferably within the hollow fibers. Subsequently, the plasma can optionally be led through another plasma filter which is used as a safety filter before it is returned to the extracorporeal blood circuit.
**Figure 3** shows an embodiment of the method according to the invention which uses a device with two pumps, three plasma filters as well as sampling ports and heating devices.
**Figure 4** shows the results of measuring the phagocytosis and oxyburst acitivity of the granulocytes used in the below Examples.
**Figure 5** shows the results of measuring the viability of the granulocytes used in the below Examples.
**Figure 6** shows the results of measuring the concentration of free haemoglobin (fHb) as a measure of cell damage.
**Figure 7** shows the results of measuring the concentration of lactate dehydrogenase (LDH) as a measure of cell damage.
**Figure 8** shows the results of measuring the concentration of monocyte chemoattractant protein-1 (MCP-1) secreted by the granulocytes.
**Figure 9** shows the results of measuring the concentration of interleukin-8 (IL-8) secreted by the granulocytes.

### EXAMPLES

The present invention is further described in more detail by the following examples which are only provided for illustrating the invention and which are not to be construed as limiting the scope of the invention. The following material and methods were used in the Examples.

### Example 1: Preparation of purified granulocyte concentrates

A standard granulocyte concentrate is prepared according to international guidelines, e.g. those described in EDQM Guide to the preparation, use and quality assurance of blood components, 20th Edition. Purified granulocyte concentrates were then prepared as described in WO 2013/182311 A1. The standard granulocyte concentrate is first sedimented until a separation line has formed between the sedimented erythrocytes and a leucocyte supernatant. Subsequently, the leucocyte supernatant is separated from the sediment, and the leucocyte supernatant is collected in a leucocyte container. The leukocytes are then sedimented in the leukocyte container by centrifugation, thereby forming a low-leukocyte supernatant. The low-leukocyte supernatant is removed from the leukocyte container. The sediment in the leukocyte container is then washed with a saline solution. The sediment in the leukocyte container is finally resuspended in a storage solution, such as human plasma. The resuspended cells are stored in a gas permeable blood bag.

### Example 2: In vitro plasma perfusion

The purified granulocyte concentrate (pGC) obtained in Example 1 was subsequently used in a preclinical *in vitro* model device for septic shock treatment. The device consisted of the following materials:
- an apheresis device (AFERsmart, Medica S.p.A., Medolla, Italy),
- blood warmers (Astotherm Plus and Astoflow Plus Eco, Stihler Electronik GmbH, Leinfelden-Echterdingen, Germany),
- a sterile, disposable tubing set (Meise GmbH Medizintechnik, Schalksmühle, Germany),
- sterile plasma filters SepaPlas 06 (Medica S.p.A., Medolla, Italy),

The disposable tubing set and the plasma filters are connected according to Figure 3 to form a closed, sterile system. This system is filled and rinsed with a hemofiltration fluid (MultiBic 4 mmol/L potassium, Fresenius Medical Care, Bad Homburg, Germany). The composition of the hemofiltration solution used for rinsing is as follows:

**Table 1: Composition of the hemofiltration solution**

| | |
|---|---|
| K⁺: | 4.0 mmol/L |
| Na⁺: | 140 mmol/L |
| Ca²⁺: | 1.5 mmol/L |
| Mg²⁺: | 0.5 mmol/L |
| Cl⁻: | 111 mmol/L |
| HCO₃⁻: | 35 mmol/L |
| Glucose: | 5.55 mmol/L |

Heparin was added as an anticoagulant in a concentration of 5 IU/mL. After rinsing the assembled tubing/filter system, the pGC preparation was heparinized with 10 IU/mL and subsequently filled into the plasma part of the tubing system. The rinsing solution was displaced by the volume of the cell preparation (approx. 400-450 mL). The cells remained inside the hollow fibres of a plasma filter (PF CC1) during the treatment. The system was then connected to a plasma pool of 1000 mL made from fresh frozen donor plasma (FFP) which represents the patient or blood sample. The plasma pool was adjusted to 20 IU/mL heparin and 1.6-2.0 mmol/L free Ca²⁺ ions. The intended concentration of free Ca²⁺ ions in the extracorporeal circulation was >1.0 mmol/L. Since the pGC cell preparation contains sodium citrate as anticoagulant and therefore almost no free Ca²⁺ ions, the initial value in the plasma pool was set correspondingly higher to reach the goal of >1.0 mmol/L after mixing. The glucose concentration was set to 3.5 - 7.0 mmol/L. To create a reservoir of oxygen in the pool bag, 1000 mL of filtered ambient air was added to the plasma pool. The plasma pool was kept at 37°C in the water bath until it was connected to the system. The total fluid volume including the plasma pool was about 1850 mL. The different components mixed completely in the first 30 minutes of the treatment simulation. The plasma perfusion treatment was continuously performed for 6 hours. Samples from the plasma stream were taken before and after the cell filter PF CC1 every hour. After 3 and 6 hours the cell filter was flushed back to obtain cells for analysis.

The samples were analyzed for the following parameters:
1. Granulocyte function: the function of the granulocytes was analysed with oxidative burst and phagocytosis assays using the commercial Phagoburst-Kit and Phagotest-Kit (Celonic, Heidelberg, Germany), respectively. Both tests were used according to the manufacturer's instructions with one modification, because the granulocyte concentration in GC is approximately 10 times higher than in whole blood. To achieve a concentration of approx. 5000 granulocytes/µL and therefore the same ratio of granulocytes to the stimulus (e.g. *Escherichia coli*) like with heparin-anticoagulated blood (4000-10,000 granulocytes/µL), the samples were diluted in heparin-anticoagulated blood group compatible plasma of healthy donors.
2. Cytokine concentrations: the measurement of cytokine concentrations was performed by use of the LEGENDplex^{™} Human Essential Immune Response Panel (13-plex). The panel is a bead-based multiplex assay panel that uses fluorescence-encoded beads suitable for use on various flow cytometers. It allows for simultaneous quantification of 13 key targets essential for immune response such as IL-4, IL-2, CXCL10 (IP-10), IL-1β, TNF-α, CCL2 (MCP-1), IL-17A, IL-6, IL-10, IFN-γ, IL-12p70, CXCL8 (IL-8), and Free Active TGF-β1. The immunoassay was performed according to the manufacturer's instructions. The quantitative analysis was carried out by a flow cytometer (MACS Quant 16, Miltenyi Biotec) and the analysis was performed using the LEGENDplex v8.0 software.
3. Blood cell counts and viability: blood cell content and leukocyte differentiation were evaluated automatically using a haematology analyser (KX-21N, Sysmex, Norderstedt, Germany). Leukocyte viability was determined using the NucleoCounter NC-200 (ChemoMetec, Allerod, Denmark) without lysing the samples.
4. Measurement of lactate, fHb and LDH: the concentrations of lactate, free haemoglobin (fHb) and lactate dehydrogenase (LDH) were determined using a Cobas Mira Plus CQ (Roche, Ludwigsburg, Germany) according to the manufacturer's specifications. The fHb concentration was measured using the 3-wavelength method (380/415/450 nm) according to the known method of Harboe [33] on the spectral photometer Dr. Lange LS 500 (Type LPG 244) according to the manufacturer's specifications.

The values at time point 3 hours are measured in samples that were taken by drawing back a volume of 50 mL from cell filter 1 (CC1). By this method, about 4% of the original cell count were retrieved, mixed, a sample was taken, and the rest of the cells was re-introduced to cell filter 1. The values at time point 6.5 hours are measured in sample that were taken at the end of the experiment by flushing back cell filter 1 with a volume of 2000mL cold sodium chloride solution. By this method, an average of 46% of the original cell count were retrieved, mixed, a sample was taken and analysed. Therefore, the cell associated results at 3h and 6h do not necessarily represent the total cell amount.

Results: The results of the measurements are depicted in Figures 4-9. It can be taken from these figures that the cells are highly active in removing toxic or inflammatory compounds from the plasma and secreting cytokines into the plasma.

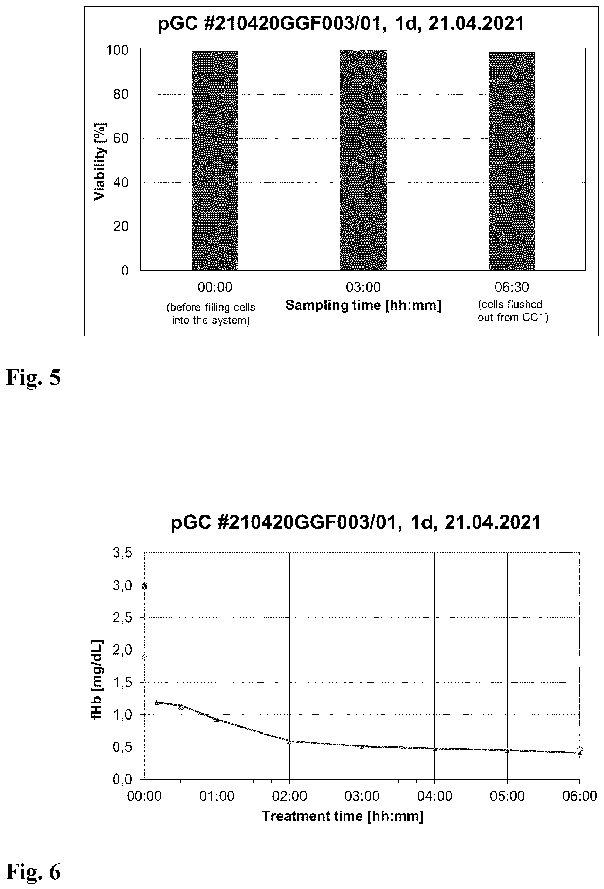

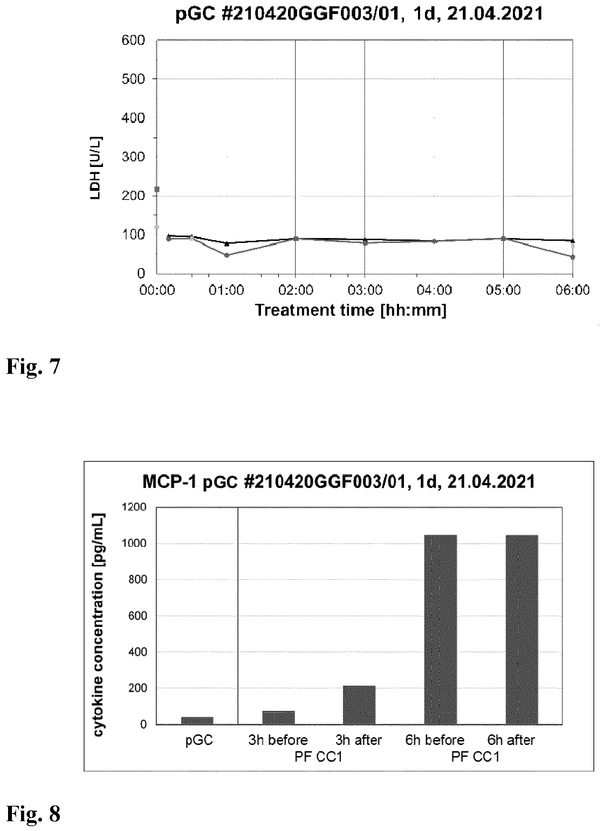

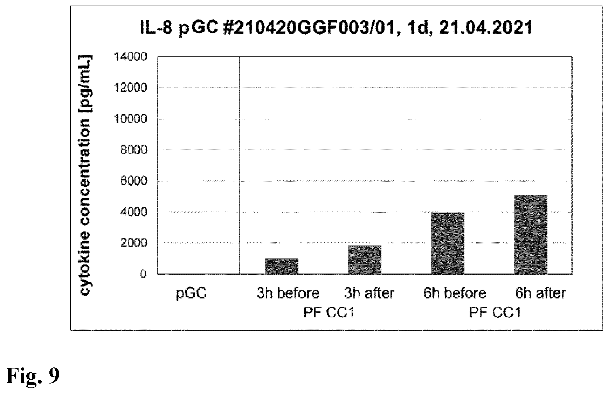

Figure 4 shows the phagocytosis rate and the oxidative burst rate of the granulocytes inside the system before, during and after the simulated treatment. The measured phagocytosis rates as well as the oxidative burst rates on day 1 were constantly higher than 80%, even after 6 hours of plasma perfusion. Figure 5 shows that the viability of the granulocytes is about 90 to 100%. Figure 6 shows the free haemoglobin concentrations as an indication for erythrocyte damage which in turn allows conclusions on the viability of the granulocytes. In view of the reference value (<10 mg/dL), all values are very low and rather decreasing during the experiment. Similarly, Figure 7 shows the LDH concentrations as an indication for cell damage. Compared to the reference value (<225 U/L), the measured values are within the physiologic range and stable during the experiment. Figures 8 and 9 show that MCP-1 and IL-8 are present in low concentrations in the pGC. The cytokines are actively secreted during the extracorporeal treatment simulation.

In summary, it follows from the experiment that the granulocytes are remain viable and active in removing toxic or inflammatory compounds from the plasma and secreting immunologically active protein into the plasma

### LITERATURE

[1] Angus, D. C., et al. (2001), Critical Care Medicine, vol. 29, no. 7, pp. 1303-1310,
[2] C. Engel, et al. (2007), Intensive Care Medicine, vol. 33, no. 4, pp. 606-618.
[3] Schmid, A. et al. (2002), Eur Journal of Health Economics, vol. 3, no. 2, pp. 77-82.
[4] Lee, H. (2004), Critical Care Medicine, vol. 32, no. 4, pp. 981-985.
[5] Takala, J. (2010), Critical Care Medicine, vol. 38, no. 10, pp. S613-S619.
[6] Kramer, L. et al. (2007), Critical Care Medicine, vol. 35, no. 4, pp. 1099-1104.
[7] Bakker, J. (2004), Critical Care Medicine, vol. 32, no. 1, pp. 1-12.
[8] Rimmelé, T. and Kellum, J. A. (2011), Critical Care, vol. 15, no. 1, article 205.
[9] Shukla, A. M. (2012), Kidney International, vol. 81, no. 4, pp. 336-338.
[10] Ellis, A. J, et al. (1996), Hepatology, vol. 24, no. 6, pp. 1446-1451.
[11] Sauer, I. M., et al. (2002), Int J Artificial Organs, vol. 25, no. 10, pp. 1001-1005.
[12] Humes, H. D. et al. (2003), Critical Care Medicine, vol. 31, no. 10, pp. 2421-2428.
[13] Humes, H. D. et al. (2003), Blood Purification, vol. 21, no. 1, pp. 64-71.
[14] Tumlin, J. et al. (2008), J Am Soc Nephrology, vol. 19, no. 5, pp. 1034-1040.
[15] Mitzner, S. R. et al. (2001), Therapeutic Apheresis, vol. 5, no. 5, pp. 423-432.
[16] Caille, V. et al. (2004), Shock, vol. 22, no. 6, pp. 521-526.
[17] Kaufmann, I. et al. (2006), Shock, vol. 26, no. 3, pp. 254-261.
[18] Pachot, A. et al. (2005), Critical Care Medicine, vol. 33, no. 1, pp. 31-38.
[19] Tavares-Murta, B. M. et al. (2002), Crit Care Med, vol. 30, no. 5, pp. 1056-1061.
[20] Keel, M. et al. (1997), Blood, vol. 90, no. 9, pp. 3356-3363.
[21] Egger, G. (2004), Intensive Care Medicine, vol. 30, no. 2, pp. 331-334.
[22] Lehmann, L. et al. (2001), Intensive Care Medicine, vol. 27, no. 8, pp.1412-1415.
[23] Wenisch, C. et al. (1995), Eur J Clin Investigation, vol. 25, no. 6, pp. 418-424.
[24] Sauer, M. et al. (2009), Critical Care Medicine, vol. 37, no. 2, pp. 606-613.
[25] Altrichter, J. et al. (2011), Critical Care, vol. 15, no. 2, article R82.
[26] Sauer, M. et al. (2009), Ther Apheresis and Dialysis, vol. 13, no. 5, pp. 444-450.
[27] Sauer M. et al. (2018), Therapeutic Apheresis and Dialysis, 22(4):389-398.
[28] Sauer M. et al. (2016), Biomed Res Int:7056492.
[29] Caille, V et al. (2004), Shock 22: 521-526, 2004.
[31] Ploder M. et al (2006), Shock 25: 129-134, 2006.
[32] Kaufmann I. et al (2006), Shock 26: 254-261, 2006.
[33] Harboe, M. (1959), Scand J Clin & Lab Invest, Vol. 11, No. 1: 66-70.

## Claims

1. *Ex vivo* method for the treatment of an immunological dysfunction of blood, said method comprising the steps of:
(a) extracorporeally separating at least part of the plasma from blood obtained from a patient suffering from an immunological dysfunction of the blood,
(b) conducting the separated plasma through a plasma-permeable hollow fiber filter comprising immune cells located thereon such that the plasma comes into contact with the immune cells, and
(c) returning the plasma obtained from step (b) to the blood.

2. *Ex vivo* method for the removal of toxic or inflammatory compounds from blood plasma and/or the supplementation of blood plasma with immunologically active proteins, said method comprising the steps of:
(a) extracorporeally separating at least part of the plasma from blood obtained from a patient suffering from an immunological dysfunction of the blood,
(b) conducting the separated plasma through a plasma-permeable hollow fiber filter comprising immune cells located thereon such that the plasma comes into contact with the immune cells, and
(c) returning the plasma obtained from step (b) to the blood.

3. The method of claim 1 or 2, wherein the immune cells comprise granulocytes, monocytes, macrophages and/or lymphocytes.

4. The method of any of claims 1-3, wherein the hollow fiber filter comprises 1 x 10¹⁰ to 1 x 10¹¹ granulocytes.

5. The method of any of claims 1-4, wherein the immunological dysfunction of the blood is associated with sepsis or a septic shock.

6. The method of any of claims 1-5, wherein less than 50%, preferably less than 10%, and more preferably less than 1% of the cells located on the hollow fiber filter are erythrocytes.

7. The method of any of claims 1-6, wherein the immune cells are located at the inner surface of the hollow fibers.

8. The method of any of claims 1-7, wherein the immune cells are derived from a healthy donor.

9. The method of any of claims 1-8, wherein the plasma-permeable hollow fiber filter has an average pore size of 0.50 µm or less.

10. The method of any of claims 1-9, wherein the method does not comprise a circulation of the immune cells.

11. The method of any of claims 1-10, wherein step (b) is conducted as a dead-end filtration.

12. The method of any of claims 1-10, wherein subsequent to step (b) and prior to step (c) the plasma is conducted through a further filter to remove any residual cells from the plasma e.g. due to a membrane rupture of said plasma-permeable hollow fiber filter.

13. Use of plasma-permeable hollow fiber filter with immune cells located thereon for
(a) the *ex vivo* treatment of an immunological dysfunction of blood,
(b) removing toxic or inflammatory compounds from blood plasma and/or supplementing blood plasma with immunologically active proteins, or
(c) producing a blood sample depleted of toxic or inflammatory compounds and/or supplemented with immunologically active proteins.

14. Use of any of claims 12-13, wherein the immune cells comprise granulocytes, monocytes, macrophages and/or lymphocytes.

15. Use of any of claims 12-14, wherein the plasma-permeable hollow fiber filter has an average pore size of 0.50 µm or less.
